(19) Europäisches Patentamt
European Patent Office
Office européen des brevets

(11) Veröffentlichungsnummer: **0 679 648 A1**

# (12) EUROPÄISCHE PATENTANMELDUNG

(21) Anmeldenummer: **95104638.2**

(22) Anmeldetag: **29.03.95**

(51) Int. Cl.[6]: **C07D 249/08**, C07D 233/61, C07D 405/06, A01N 43/653, A01N 43/50

(30) Priorität: **11.04.94 DE 4412358**

(43) Veröffentlichungstag der Anmeldung: **02.11.95 Patentblatt 95/44**

(84) Benannte Vertragsstaaten: **BE CH DE ES FR GB IT LI NL**

(71) Anmelder: **BAYER AG**
**D-51368 Leverkusen (DE)**

(72) Erfinder: **Jautelat, Manfred, Dr.**
**Müllersbaum 28**
**D-51399 Burscheid (DE)**
Erfinder: **Dutzmann, Stefan, Dr.**
**Kosenberg 10**
**D-40721 Hilden (DE)**
Erfinder: **Stenzel, Klaus, Dr.**
**Seesener Strasse 17**
**D-40595 Düsseldorf (DE)**
Erfinder: **Dehne, Heinz-Wilhelm, Dr.**
**Charles-Wimer-Strasse 15**
**D-53125 Bonn (DE)**

## (54) Azido-substituierte Cyclopropyl-ethyl-azole, deren Herstellung und deren Verwendung als Fungizide.

(57) Neue Cyclopropyl-ethyl-azole der Formel

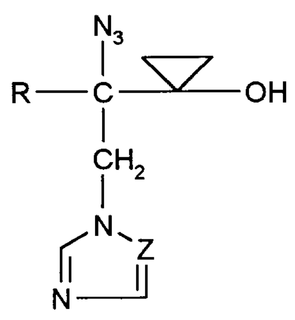

(I)

in welcher

R für Alkyl, Alkenyl, Alkinyl, gegebenenfalls durch Alkyl substituiertes Cycloalkyl, gegebenenfalls substituiertes Aryl, gegebenenfalls substituiertes Aralkyl oder gegebenenfalls substituiertes Aralkenyl steht und

Z für ein Stickstoffatom oder eine CH-Gruppe steht,

sowie deren Säureadditions-Salze und Metallsalz-Komplexe,
ein Verfahren zur Herstellung der neuen Stoffe und deren Verwendung als Fungizide.

Neue Oxirane der Formel

EP 0 679 648 A1

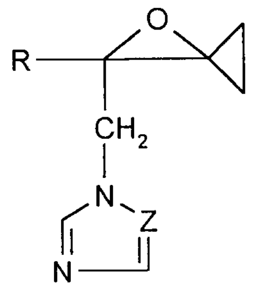

(II)

in welcher
R und Z die oben angegebenen Bedeutungen haben,
ein Verfahren zur Herstellung dieser Oxirane und deren Verwendung als Zwischenprodukte.

Die vorliegende Erfindung betrifft neue Cyclopropyl-ethyl-azole, ein Verfahren zu deren Herstellung und deren Verwendung als Fungizide. Außerdem betrifft die Erfindung neue Zwischenprodukte und ein Verfahren zu deren Herstellung.

Es ist bereits bekannt geworden, daß bestimmte Hydroxyethyl-azolyl-Derivate fungizide Eigenschaften besitzen (vgl. EP-OS 0 438 686 und EP-OS 0 123 160). So lassen sich zum Beispiel 1-(2-Chlorphenyl)-2-(1-methoxy-cycloprop-1-yl)-3-(1,2,4-triazol-1-yl)-propan-2-ol und 1-(2-Chlorphenyl)-2-(1,2,4-triazol-1-yl-methyl)-3,3-dimethyl-butan-2-ol zur Bekämpfung von Pilzen verwenden. Die Wirkung dieser Stoffe ist gut, läßt aber bei niedrigen Aufwandmengen in manchen Fällen zu wünschen übrig.

Es wurden nun neue Cyclopropyl-ethyl-azole der Formel

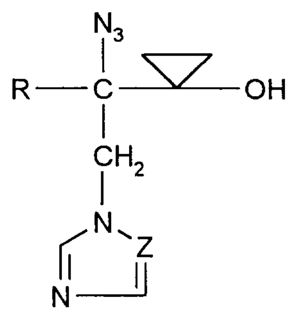

(I)

in welcher

R für Alkyl, Alkenyl, Alkinyl, gegebenenfalls durch Alkyl substituiertes Cycloalkyl, gegebenenfalls substituiertes Aryl, gegebenenfalls substituiertes Aralkyl oder gegebenenfalls substituiertes Aralkenyl steht und

Z für ein Stickstoffatom oder eine CH-Gruppe steht,

sowie deren Säureadditions-Salze und Metallsalz-Komplexe gefunden.

Die erfindungsgemäßen Stoffe enthalten ein asymmetrisch substituiertes Kohlenstoffatom. Sie können daher in optischen Isomerenformen anfallen. Die vorliegende Erfindung betrifft sowohl die einzelnen Isomeren als auch deren Gemische.

Weiterhin wurde gefunden, daß man Cyclopropyl-ethyl-azole der Formel (I) sowie deren Säureadditions-Salze und Metallsalz-Komplexe erhält, wenn man Oxirane der Formel

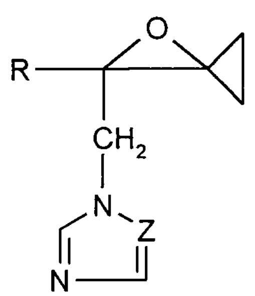

(II)

in welcher

R und Z die oben angegebenen Bedeutungen haben,

mit Natriumazid in Gegenwart eines zusätzlichen Verdünnungsmittels sowie gegebenenfalls in Gegenwart einer Base umsetzt und unter Zugabe von Wasser aufarbeitet,

und gegebenenfalls anschließend an die so erhaltenen Verbindungen der Formel (I) eine Säure oder ein Metallsalz addiert.

Schließlich wurde gefunden, daß die neuen Cyclopropyl-ethyl-azole der Formel (I) sowie deren Säureadditions-Salze und Metallsalz-Komplexe sehr gute fungizide Eigenschaften aufweisen.

Überraschenderweise zeigen die erfindungsgemäßen Stoffe eine bessere fungizide Wirksamkeit als die konstitutionell ähnlichsten vorbekannten Verbindungen gleicher Wirkungsrichtung.

Die erfindungsgemäßen Cyclopropyl-ethyl-azole sind durch die Formel (I) allgemein definiert.

R steht vorzugsweise für geradkettiges oder verzweigtes Alkyl mit 1 bis 8 Kohlenstoffatomen, geradkettiges oder verzweigtes Alkenyl mit 2 bis 8 Kohlenstoffatomen, geradkettiges oder verzweigtes Alkinyl mit 2 bis 8 Kohlenstoffatomen, oder

für Cycloalkyl mit 3 bis 7 Kohlenstoffatomen, wobei jeder dieser Cycloalkylreste einfach bis dreifach, gleichartig oder verschieden substituiert sein kann durch Alkyl mit 1 bis 4 Kohlenstoffatomen, oder

für Phenyl, das einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano, oder

für Phenylalkyl mit 1 bis 4 Kohlenstoffatomen im geradkettigen oder verzweigten Alkylteil, wobei der Phenylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mti 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano, oder

für Phenylalkenyl mit 2 bis 4 Kohlenstoffatomen im geradkettigen oder verzweigten Alkenylteil, wobei der Phenylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano.

Z steht auch vorzugsweise für Stickstoff oder eine CH-Gruppe.

R steht besonders bevorzugt für geradkettiges oder verzweigtes Alkyl mit 1 bis 6 Kohlenstoffatomen, geradkettiges oder verzweigtes Alkenyl mit 2 bis 6 Kohlenstoffatomen, geradkettiges oder verzweigtes Alkinyl mit 2 bis 6 Kohlenstoffatomen, oder

für Cycloalkyl mit 3 bis 7 Kohlenstoffatomen, wobei jeder dieser Reste einfach bis dreifach, gleichartig oder verschieden substituiert sein kann durch Methyl und/oder Ethyl, oder

für Phenyl, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Methoxycarbonyl, Ethoxycarbonyl, Methoximinomethyl, 1-Methoximinoethyl, Nitro und/oder Cyano, oder

für Phenylalkyl mit 1 bis 3 Kohlenstoffatomen im geradkettigen oder verzweigten Alkylteil, wobei der Phenylteil jeweils einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Methoxycarbonyl, Ethoxycarbonyl, Methoximinomethyl, 1-Methoximinoethyl, Nitro und/oder Cyano, oder

für Phenylalkenyl mit 2 bis 4 Kohlenstoffatomen im geradkettigen oder verzweigten Alkenylteil, wobei der Phenylteil jeweils einfach bis dreifach, gleichartig oder verschieden substituiert sein kann durch Fluor, Chlor, Brom, Methyl, Ethyl, tert.-Butyl, Methoxy, Ethoxy, Methylthio, Trifluomethyl, Trifluormethoxy, Trifluormethylthio, Chlordifluormethoxy, Chlordifluormethylthio, Methoxycarbonyl, Ethoxycarbonyl, Methoximinomethyl, 1-Methoximinoethyl, Nitro und/oder Cyano.

Z steht auch besonders bevorzugt für Stickstoff oder eine CH-Gruppe.

Bevorzugte erfindungsgemäße Stoffe sind auch Additionsprodukte aus Säuren und Cyclopropyl-ethylazolen der Formel (I), in denen R und Z die oben als bevorzugt genannten Bedeutungen haben.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure, und außerdem auch Saccharin und Thiosaccharin.

Außerdem bevorzugte erfindungsgemäße Stoffe sind Additionsprodukte aus Salzen von Metallen der II. bis IV. Haupt- und der I. und II. sowie IV. bis VIII. Nebengruppe des Periodensystems der Elemente und Cyclopropyl-ethyl-azolen der Formel (I), in denen R und Z die oben als bevorzugt genannten Bedeutungen haben.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. Chlorwasserstoffsäure und die Bromwasserstoffsäure, ferner Phosphorsäure, Salpetersäure und Schwefelsäure.

Als Beispiele für erfindungsgemäße Stoffe seien die in der folgenden Tabelle aufgeführten Cyclopropylethyl-azole genannt.

**Tabelle 1**

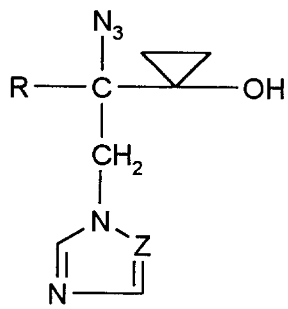

(I)

| R | Z |
|---|---|
| $-C_4H_9$-n | N |
| $-C_4H_9$-t | N |
| $-CH_2-CH=CH_2$ | N |
| $-C_4H_9$-n | CH |
| $-C_4H_9$-t | CH |
| $-CH_2-CH=CH-CH_3$ | N |
| $-CH_2-C\equiv CH$ | N |
| 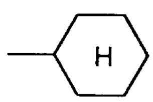 | N |
| 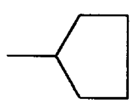 | N |
| 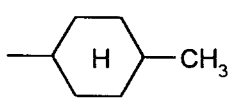 | N |
| 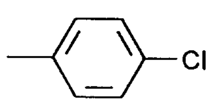 | N |

**Tabelle 1** (Fortsetzung)

| R | Z |
|---|---|
| 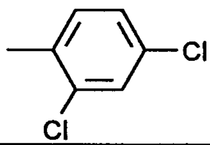 | N |
| 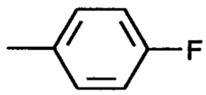 | N |
| 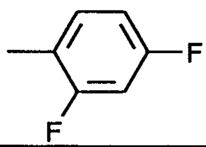 | N |
| 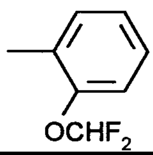 | N |
| 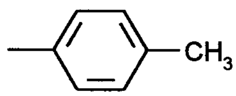 | N |
| 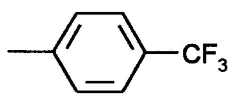 | N |
| 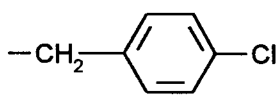 | N |
| 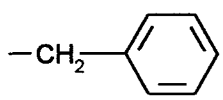 | N |
| 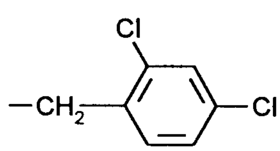 | N |
| 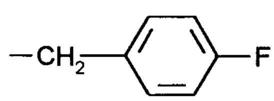 | N |

**Tabelle 1** (Fortsetzung)

| R | Z |
|---|---|
| 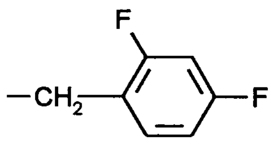 | N |
| 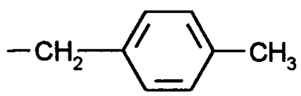 | N |
| 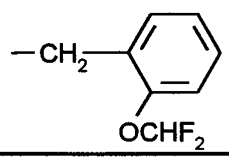 | N |
| 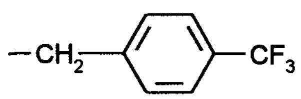 | N |
| 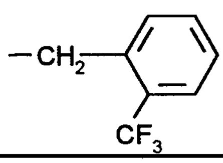 | N |
| 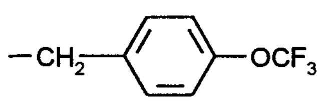 | N |
| 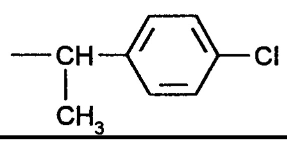 | N |
| 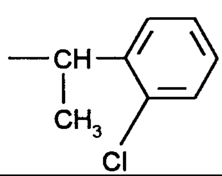 | N |
| 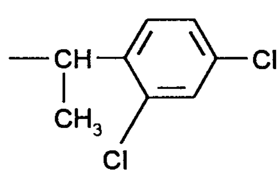 | N |

**Tabelle 1** (Fortsetzung)

| R | Z |
|---|---|
| 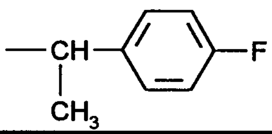 | N |
| 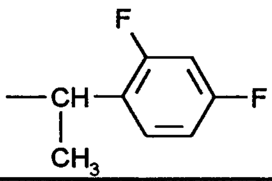 | N |
| 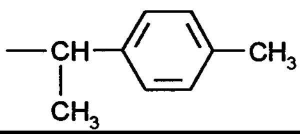 | N |
| 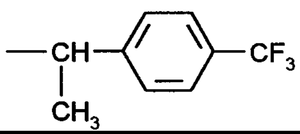 | N |
| 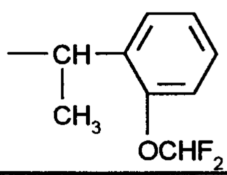 | N |
| 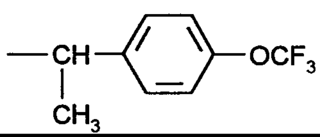 | N |
| 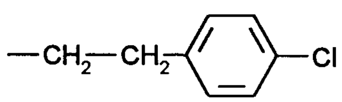 | N |
| 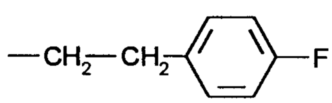 | N |
| 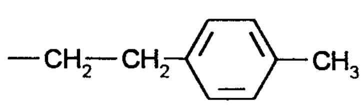 | N |

**Tabelle 1** (Fortsetzung)

| R | Z |
|---|---|
| 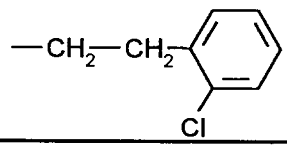 | N |
| 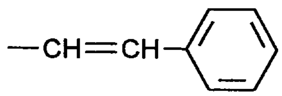 | N |
| 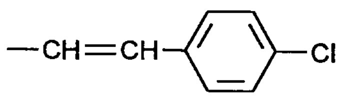 | N |
| 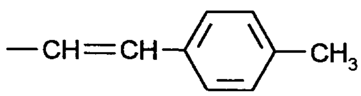 | N |
| 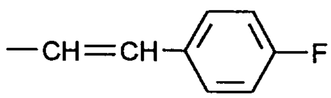 | N |
| 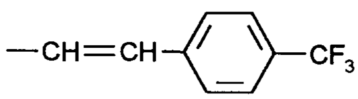 | N |
| 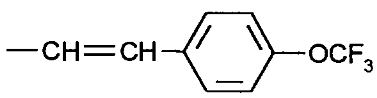 | N |
| 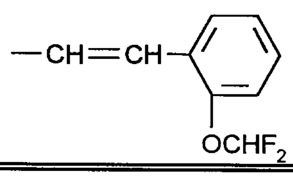 | N |

Verwendet man 3-(2-Chlorbenzyl)-3-(1,2,4-triazol-1-yl-methyl)-2-oxaspiro[2,2]pentan und Natriumazid als Ausgangsstoffe und wäßrige Natriumcarbonat-Lösung zur anschließenden Hydrolyse, so kann der Verlauf des erfindungsgemäßen Verfahrens durch das folgende Formelschema veranschaulicht werden:

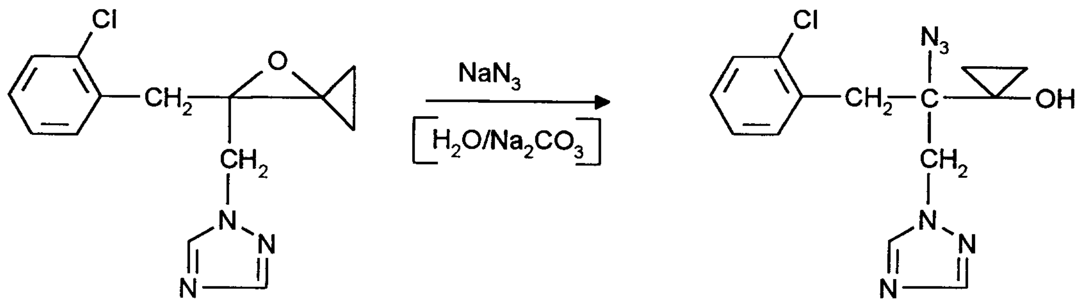


Die bei der Durchführung des erfindungsgemäßen Verfahrens als Ausgangsstoffe benötigten Oxirane sind durch die Formel (II) allgemein definiert. In dieser Formel haben R und Z vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele für Oxirane der Formel (II) seien die in der folgenden Tabelle 2 aufgeführten Stoffe genannt.

**Tabelle 2**

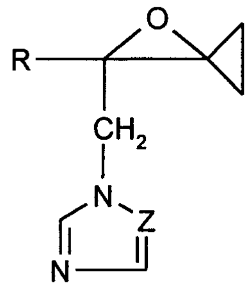

(II)

| R | Z |
|---|---|
| $-C_4H_9$-n | N |
| $-C_4H_9$-t | N |
| $-CH_2-CH = CH_2$ | N |
| $-C_4H_9$-n | CH |
| $-C_4H_9$-t | CH |
| $-CH_2-CH = CH-CH_3$ | N |
| $-CH_2-C \equiv CH$ | N |
| 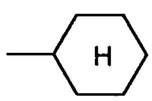 | N |
| 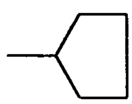 | N |
| 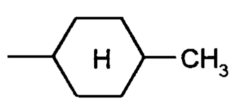 | N |
| 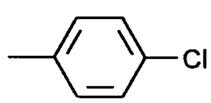 | N |

**Tabelle 2** (Fortsetzung)

| R | Z |
|---|---|
| 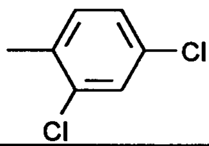 | N |
| 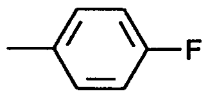 | N |
| 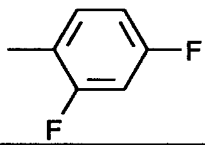 | N |
| 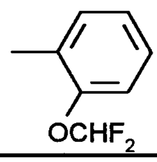 | N |
| 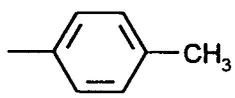 | N |
| 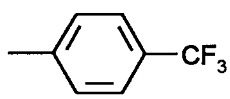 | N |
| 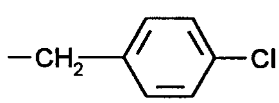 | N |
| 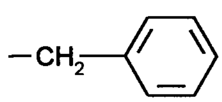 | N |
| 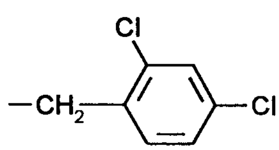 | N |
| 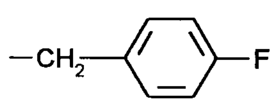 | N |

**Tabelle 2** (Fortsetzung)

| R | Z |
|---|---|
| 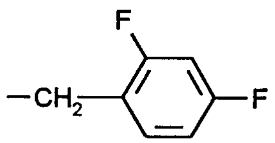 | N |
| 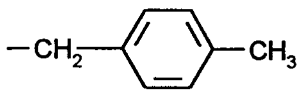 | N |
| 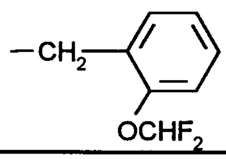 | N |
| 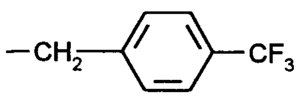 | N |
| 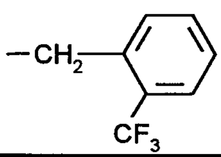 | N |
| 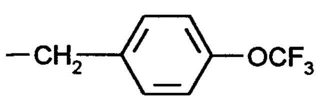 | N |
| 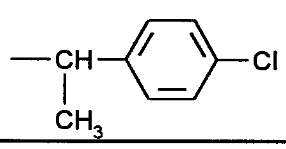 | N |
| 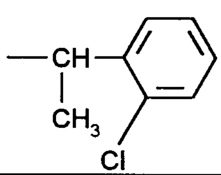 | N |
| 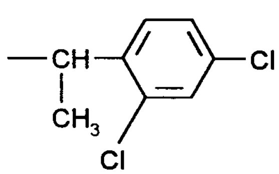 | N |

**Tabelle 2** (Fortsetzung)

| R | Z |
|---|---|
| 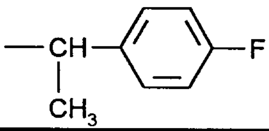 | N |
| 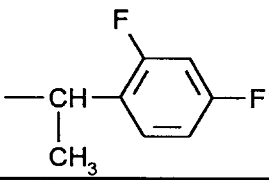 | N |
| 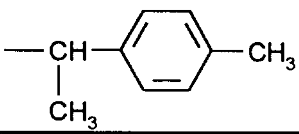 | N |
| 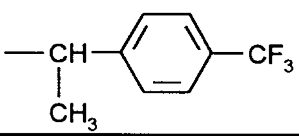 | N |
| 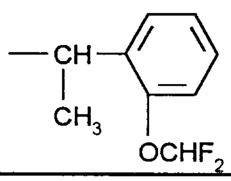 | N |
| 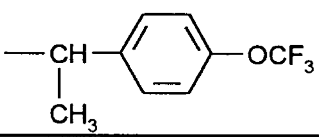 | N |
| 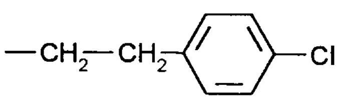 | N |
| 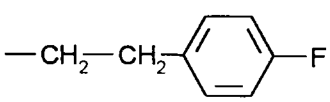 | N |
| 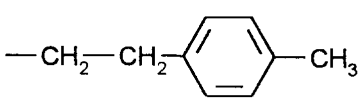 | N |

**Tabelle 2** (Fortsetzung)

| R | Z |
|---|---|
| 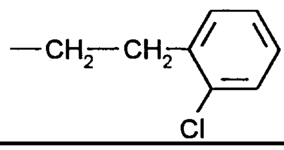 | N |
| 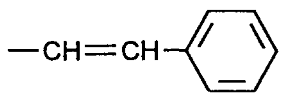 | N |
| 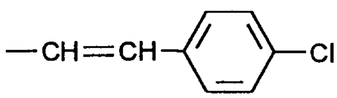 | N |
| 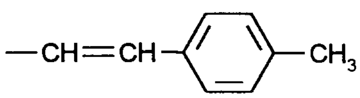 | N |
| 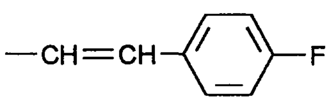 | N |
| 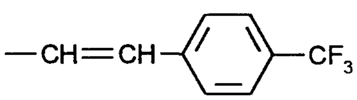 | N |
| 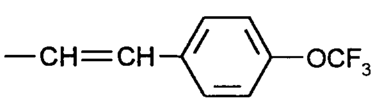 | N |
| 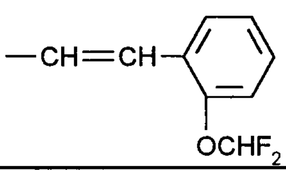 | N |

Die Oxirane der Formel (II) sind bisher noch nicht bekannt. Sie lassen sich herstellen, indem man Cyclopropyl-hydroxyethyl-azole der Formel

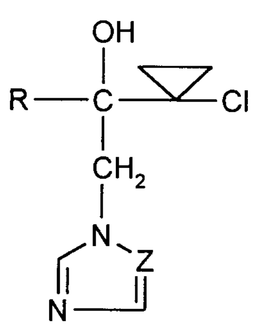

(III)

in welcher
R und Z die oben angegebenen Bedeutungen haben,
in Gegenwart von starken Basen und in Gegenwart eines Verdünnungsmittels umsetzt.

Verwendet man 2-(1-Chlorcyclopropyl)-1-(2-chlorphenyl)-2-hydroxy-3-(1,2,4-triazol-1-yl)-propan als Ausgangsstoff und Kalium-tert.-butylat als starke Base, so kann der Verlauf des Verfahrens zur Herstellung von Oxiranen der Formel (II) durch das folgende Formelschema veranschaulicht werden:

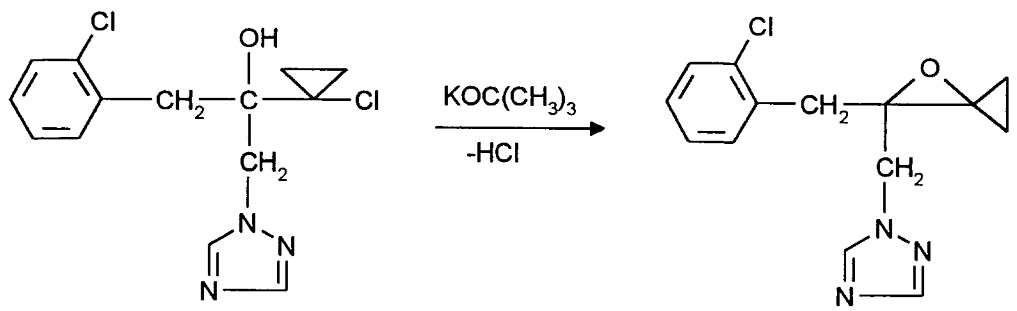


Die bei der Herstellung von Oxiranen der Formel (II) als Ausgangsstoffe benötigten Cyclopropyl-hydroxyethyl-azole sind durch die Formel (III) allgemein definiert. In dieser Formel haben R und Z vorzugsweise diejenigen Bedeutungen, die bereits im Zusammenhang mit der Beschreibung der erfindungsgemäßen Stoffe der Formel (I) vorzugsweise für diese Reste genannt wurden.

Die Cyclopropyl-hydroxyethyl-azole der Formel (III) sind bekannt oder lassen sich nach prinzipiell bekannten Methoden herstellen (vgl. EP-OS 0 180 136, EP-OS 0 297 345, EP-OS 0 297 383, EP-OS 0 298 332, EP-OS 0 440 949 und EP-OS 0 470 463).

Als Basen kommen bei der Durchführung des Verfahrens zur Herstellung von Oxiranen der Formel (II) alle üblichen starken anorganischen und organischen Basen in Betracht. Vorzugsweise verwendbar sind Alkalimetallalkoholate, wie Natriummethylat, Natriumethylat und Kalium-tert.-butylat, und außerdem Alkalimetallhydride, wie Natriumhydrid.

Als Verdünnungsmittel kommen bei der Durchführung des Verfahrens zur Herstellung von Oxiranen der Formel (II) alle für derartige Umsetzungen üblichen, inerten organischen Solventien in Frage. Vorzugsweise verwendbar sind Alkohole, wie Methanol, Ethanol, Propanol und tert.-Butanol, weiterhin Ether, wie Diethylether, 1,2-Dimethoxyethan, Tetrahydrofuran und Dioxan, und außerdem aliphatische, cycloaliphatische und aromatische Kohlenwasserstoffe, wie Pentan, Hexan, Cyclohexan, Benzol, Toluol und Xylol.

Die Reaktionstemperaturen können bei der Durchführung des Verfahrens zur Herstellung von Oxiranen der Formel (II) innerhalb eines größeren Bereichs variiert werden. Im allgemeinen arbeitet man bei Temperaturen zwischen 0°C und 100°C, vorzugsweise zwischen 20°C und 80°C.

Bei der Durchführung des Verfahrens zur Herstellung von Oxiranen der Formel (II) arbeitet man im allgemeinen unter Normaldruck. Es ist aber auch möglich, unter erhöhtem oder vermindertem Druck zu arbeiten.

Bei der Durchführung des Verfahrens zur Herstellung von Oxiranen der Formel (II) setzt man auf 1 Mol an Cyclopropyl-hydroxyethyl-azol der Formel (III) im allgemeinen 1 bis 1,5 Äquivalente an starker Base ein. Die Aufarbeitung erfolgt nach üblichen Methoden. Im allgemeinen geht man in der Weise vor, daß man das Reaktionsgemisch, gegebenenfalls nach vorherigem Verdünnen mit einem mit Wasser wenig mischbaren organischen Lösungsmittel, mit Wasser wäscht, die organische Phase trocknet und einengt und das verbleibende Produkt gegebenenfalls nach üblichen Methoden, wie z.B. durch Chromatographie, von eventuell vorhandenen Verunreinigungen befreit.

Bei der Durchführung des erfindungsgemäßen Verfahrens zur Herstellung von Cyclopropyl-ethyl-azolen der Formel (I) dient Natriumazid als Reaktionskomponente. Wasser wird erst bei der Aufarbeitung hinzugefügt.

Als Verdünnungsmittel kommen bei der Durchführung des erfindungsgemäßen Verfahrens zur Herstellung von Stoffen der Formel (I) polare, aprotische organische Solventien in Betracht. Vorzugsweise verwendbar sind Nitrile, wie Acetonitril und Propionitril, weiterhin Amide, wie Dimethylformamid und N-Methyl-pyrrolidon, und außerdem stark polare Solventien, wie Dimethylsulfoxid.

Als Basen kommen bei der Durchführung des erfindungsgemäßen Verfahrens zur Herstellung von Stoffen der Formel (I) alle üblicherweise für derartige Umsetzungen geeigneten anorganischen und organischen Säurebindemittel in Frage. Vorzugsweise verwendbar sind Alkalimetallcarbonate, wie Natriumcarbonat und Kaliumcarbonat, und außerdem Ammoniumcarbonat, und ferner teriäre Amine, wie Triethylamin und Pyridin.

Die Reaktionstemperaturen können bei der Durchführung des erfindungsgemäßen Verfahrens zur Herstellung von Stoffen der Formel (I) in einem größeren Bereich variiert werden. Im allgemeinen arbeitet man bei Temperaturen zwischen 20 °C und 130 °C, vorzugsweise zwischen 30 °C und 100 °C.

Bei der Durchführung des erfindungsgemäßen Verfahrens arbeitet man im allgemeinen unter Normaldruck. Es ist aber auch möglich, unter erhöhtem oder vermindertem Druck zu arbeiten.

Bei der Durchführung des erfindungsgemäßen Verfahrens setzt man auf 1 Mol an Oxiran der Formel (II) im allgemeinen 1 Mol an Natriumazid oder auch einen Überschuß ein. Die Aufarbeitung erfolgt nach üblichen Methoden. Im allgemeinen verfährt man in der Weise, daß man das Reaktionsgemisch, gegebenenfalls nach vorherigem Verdünnen mit einem mit Wasser wenig mischbaren organischen Solvens, mit Wasser gegebenenfalls in Gegenwart einer Alkalimetall-Base wäscht, die organische Phase trocknet und einengt und das verbleibende Produkt gegebenenfalls nach üblichen Methoden, z.B. durch Chromatographie, von eventuell vorhandenen Verunreinigungen befreit.

Die erfindungsgemäßen Cyclopropyl-ethyl-azole der Formel (I) können in Säureadditions-Salze oder Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubensis;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;

Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Getreidekrankheiten, wie Erysiphe, Leptosphaeria und Fusarium, sowie zur Bekämpfung von Plasmopara, Venturia und Podosphaera im Obst-, Wein- und Gemüsebau. Sie können außerdem eingesetzt werden gegen Reiskrankheiten, wie Pyricularia oryzae, und besitzen auch eine gute und breite in-vitro-Wirkung.

Die erfindungsgemäßen Stoffe können in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mittels, also Emulgiermitteln und/oder Dispergiermitteln und/oder schaumerzeugenden Mitteln. Im Falle der Benutzung von Wasser als Streckmittel können z.B. auch organische Lösungsmittel wie Alkohole als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe wie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff vorzugsweise zwischen 0,5 und 90 %.

Die erfindungsgemäßen Wirkstoffe können in den Formulierungen in Mischung mit bekannten Fungiziden, Bakteriziden, Akariziden, Nematiziden oder Insektiziden eingesetzt werden, um so z.B. das Wirkungsspektrum zu verbreitern oder Resistenzentwicklungen vorzubeugen. In manchen Fällen tritt auch Synergismus auf.

Für die Mischungen kommen beispielsweise folgende Stoffe in Frage.

**Fungizide:**

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoximino-N-methyl-2-(2-phenoxyphenyl)-acetamid; 8-Hydroxychinolinsulfat; Methyl-(E)-2-{2-[6-(2-cyanophenoxy)-pyrimidin-4-yloxy]-phenyl}-3-methoxyacrylat; Methyl-(E)-methoximino [alpha-(o-tolyloxy)-o-tolyl]-acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP) Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil, Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

**Bakterizide:**

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

**Insektizide / Akarizide / Nematizide:**

Abamectin, AC 303 630, Acephat, Acrinathrin, Alanycarb, Aldicarb, Alpha-methrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,

HCH, Heptenophos, Hexaflumuron, Hexythiazox,
Imidacloprid, Iprobenfos, Isazophos, Isofenphos, Isoprocarb, Isoxathion, Ivemectin, Lambda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184 NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.

Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.
Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die Herstellung und die Verwendung von erfindungsgemäßen Wirkstoffen werden durch die folgenden Beispiele veranschaulicht.

## Herstellungsbeispiele

### Beispiel 1

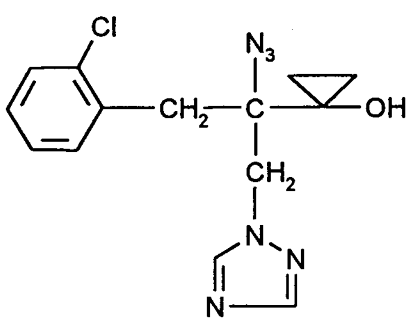

(I-1)

Ein Gemisch aus 2,76 g (10 mMol) 3-(2-Chlorbenzyl)-3-(1,2,4-triazol-1-yl-methyl)-2-oxa-spiro[2,2]pentan, 0,39 g (6 mMol) Natriumazid und 20 ml absolutem Dimethylformamid wird 3 Stunden unter Rühren auf 80 °C erhitzt. Danach wird das Reaktionsgemisch mit Ethylacetat versetzt und mehrfach mit gesättigter, wäßriger Natriumcarbonat-Lösung ausgeschüttelt. Die organische Phase wird über Natriumsulfat getrocknet und unter vermindertem Druck eingeengt. Man erhält 3,3 g eines Rohproduktes, das mit Ethylacetat als

Laufmittel über 300 g Kieselgel chromatographiert wird. Nach dem Einengen des Eluates erhält man 1,3 g (41 % der Theorie) an 2-Azido-2-(1-hydroxy-cyclopropyl-1-yl)-1-(2-chlorphenyl)-3-(1,2,4-triazol-1-yl)-propan in Form einer Feststubstanz vom Schmelzpunkt 137-139 °C.

## Herstellung von Ausgangssubstanzen

### Beispiel 2

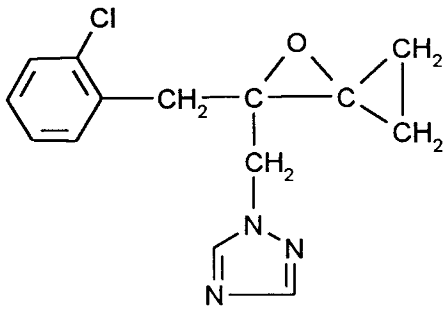

(II-1)

Ein Gemisch aus 3,12 g (10 mMol) 2-(1-Chlorcyclopropyl)-1-(2-chlorphenyl)-2-hydroxy-3-(1,2,4-triazol-1-yl)-propan, 1,12 g (10 mMol) Kalium-tert.-butylat und 50 ml absolutem tert.-Butanol wird 25 Stunden bei 60 °C gerührt. Danach wird das Reaktionsgemisch mit Ethylacetat verdünnt und mehrfach mit Wasser gewaschen. Die organische Phase wird über Natriumsulfat getrocknet und unter vermindertem Druck eingeengt. Man erhält 3,29 g eines Rohrproduktes, das mit Ethylacetat als Laufmittel über 300 g Kieselgel chromatographiert wird. Nach dem Einengen des Eluates erhält man 1,3 g (47 % der Theorie) an 3-(2-Chlorbenzyl)-3-(1,2,4-triazol-1-yl-methyl)-2-oxa-spiro[2,2]-pentan in Form eines Öles.
$^1$H-NMR-Spektrum (200 MHz, $CDCl_3$, TMS):
δ = 0,7-0,95 (m, 4H); 3,3 (2, 2H); 4,55 (AB, 2H); 7,2-7,4 (m, 4H); 7,9 (s, 1H); 8,1 (s, 1H) ppm
GC/MS(Ci): 276 (M + $H^+$, 100 %)

### Beispiel 3

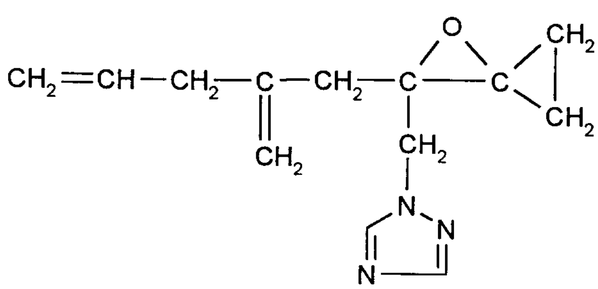

(II-2)

Ein Gemisch aus 1,34 g (5 mMol) 6-(1-Chlorcyclopropyl)-6-hydroxy-4-methylen-7-(1,2,4-triazol-1-yl)-hepten-1, 0,56 g (5 mMol) Kalium-tert.-butylat und 30 ml absolutem tert.-Butanol wird 22 Stunden bei 60 °C gerührt. Danach wird das Reaktionsgemisch mit Ethylacetat verdünnt und mehrfach mit Wasser gewaschen. Die organische Phase wird über Natriumsulfat getrocknet und unter vermindertem Druck eingeengt. Man erhält 1,1 g eines Rohproduktes, das mit Ethylacetat als Laufmittel über 300 g Kieselgel chromatographiert wird. Nach dem Einengen des Eluates erhält man 0,8 g (70 % der Theorie) an 3-(2-Methylen-pent-4-enyl)-3-(1,2,4-triazol-1-yl-methyl)-2-oxa-spiro[2,2]pentan in Form eines Öles.
$^1$H-NMR-Spektrum (200 MHz, $CDCl_3$, TMS):
δ (ppm) = 0,8-1,1 (m, 4H); 2,45 (AB, 2H); 2,7 (AB, 2H); 4,55 (s, 2H); 5.0 (m, 4H); 5,8 (m, 1H); 7,95 (s, 1H); 8,15 (s, 1H)
GC/MS(Ci): 232 (M + $H^+$, 100 %)

**Beispiel 4**

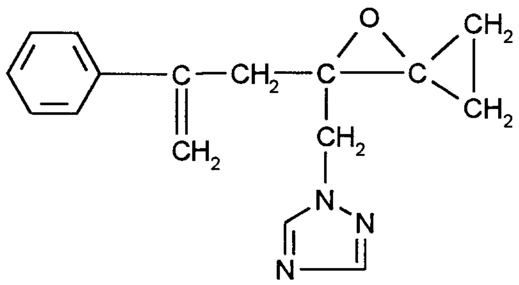

(II-3)

Ein Gemisch aus 1,52 g (5 mMol) 4-(1-Chlor-cyclopropyl)-4-hydroxy-2-phenyl-5-(1,2,4-triazol-1-yl)-penten-1, 0,67 g (6 mMol) Kalium-tert.-butylat und 50 ml absolutem Tetrahydrofuran wird 5 Stunden bei 40 °C gerührt. Danach wird das Reaktionsgemisch mit Ethylacetat verdünnt und mehrfach mit gesättigter, wäßriger Natriumcarbonat-Lösung gewaschen. Die organische Phase wird über Natriumsulfat getrocknet und unter vermindertem Druck eingeengt. Man erhält 1,3 g eines Rohrproduktes, das mit Ethylacetat als Laufmittel über 200 g Kieselgel chromatographiert wird. Nach dem Einengen des Eluates erhält man 0,5 g (37 % der Theorie) an 3-(2-Phenyl-prop-1-en-3-yl)-3-(1,2,4-triazol-1-yl-methyl)-2-oxaspiro[2,2]pentan in Form eines Öles.

GC/MS(Ci): 268 (M + $H^+$, 100 %)

**Beispiel 5**

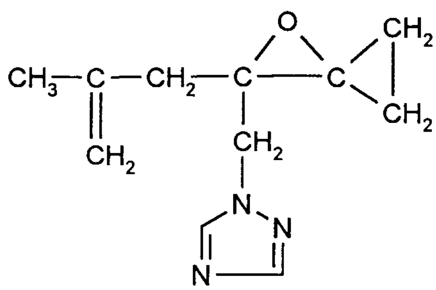

(II-4)

Nach der im Beispiel 4 angegebenen Methode wird auch die Verbindung der Formel (II-4) hergestellt. Man erhält die Substanz in Form eines Öles.

GC/MS (Ci): 206 (M + $H^+$, 100 %)

**Verwendungsbeispiele**

**Beispiel A**

Erysiphe-Test (Gerste) / protektiv

Lösungsmittel: 10 Gewichtsteile N-Methyl-pyrrolidon
Emulgator: 0,6 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Bei einer Aufwandmenge von 250 g/ha zeigt die erfindungsgemäße Verbindung (I-1) in diesem Test einen Wirkungsgrad von 100 %.

## Beispiel B

Gibberella zeae-Test (Gerste) / protektiv (syn. Fusarium graminearum)

Lösungsmittel: 10 Gewichtsteile N-Methyl-pyrrolidon
Emulgator: 0,6 Gewichtsteile Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit einer Konidiensuspension von Gibberella zeae besprüht.

Die Pflanzen werden in einem Gewächshaus unter lichtdurchlässigen Inkubationshauben bei einer Temperatur von ca. 20 °C und einer relativen Luftfeuchtigkeit von ca. 100 % aufgestellt.

4 Tage nach der Inokulation erfolgt die Auswertung.

Bei einer Aufwandmenge von 250 g/ha zeigt die erfindungsgemäße Verbindung (I-1) in diesem Test einen Wirkungsgrad von 100 %.

## Beispiel C

Plasmopara-Test (Rebe) / protektiv

Lösungsmittel: 4,7 Gewichtsteile Aceton
Emulgator: 0,3 Gewichtsteile Alkyl-Aryl-Polyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22 °C und 100 % relativer Luftfeuchtigkeit. Anschließend werden die Pflanzen 5 Tage im Gewächshaus bei 21 °C und 90% Luftfeuchtigkeit aufgestellt. Die Pflanzen werden dann angefeuchtet und 1 Tag in eine Feuchtekammer gestellt.

6 Tage nach der Inokulation erfolgt die Auswertung.

Bei einer Wirkstoffkonzentration von 10 ppm zeigt die erfindungsgemäße Verbindung (I-1) in diesem Test einen Wirkungsgrad von über 90 %.

## Patentansprüche

**1.** Cyclopropyl-ethyl-azole der Formel

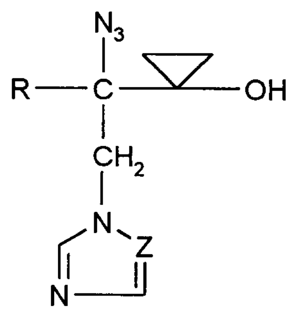

(I)

in welcher

R für Alkyl, Alkenyl, Alkinyl, gegebenenfalls durch Alkyl substituiertes Cycloalkyl, gegebenenfalls substituiertes Aryl, gegebenenfalls substituiertes Aralkyl oder gegebenenfalls substituiertes Aralkenyl steht und

Z für ein Stickstoffatom oder eine CH-Gruppe steht,

sowie deren Säureadditions-Salze und Metallsalz-Komplexe.

**2.** Cyclopropyl-ethyl-azole der Formel (I) gemäß Anspruch 1, in denen

R für geradkettiges oder verzweigtes Alkyl mit 1 bis 8 Kohlenstoffatomen, geradkettiges oder verzweigtes Alkenyl mit 2 bis 8 Kohlenstoffatomen, geradkettiges oder verzweigtes Alkinyl mit 2 bis 8 Kohlenstoffatomen, oder für Cycloalkyl mit 3 bis 7 Kohlenstoffatomen steht, wobei jeder dieser Cycloalkylreste einfach bis dreifach, gleichartig oder verschieden substituiert sein kann durch Alkyl mit 1 bis 4 Kohlenstoffatomen,

oder für Phenyl steht, das einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano, oder

für Phenylalkyl mit 1 bis 4 Kohlenstoffatomen im geradkettigen oder verzweigten Alkylteil steht, wobei der Phenylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mti 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano, oder

für Phenylalkenyl mit 2 bis 4 Kohlenstoffatomen im geradkettigen oder verzweigten Alkenylteil steht, wobei der Phenylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano und

Z für Stickstoff oder eine CH-Gruppe steht.

**3.** Verfahren zur Herstellung von Cyclopropyl-ethyl-azolen der Formel

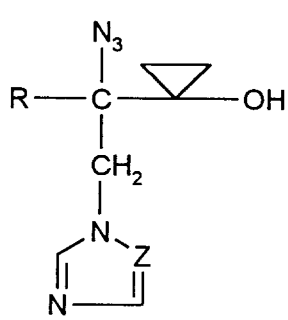

(I)

in welcher

R für Alkyl, Alkenyl, Alkinyl, gegebenenfalls durch Alkyl substituiertes Cycloalkyl, gegebenenfalls substituiertes Aryl, gegebenenfalls substituiertes Aralkyl oder gegebenenfalls substituiertes Aralkenyl steht und

Z für ein Stickstoffatom oder eine CH-Gruppe steht,

sowie von deren Säureadditions-Salzen und Metallsalz-Komplexen, dadurch gekennzeichnet, daß man Oxirane der Formel

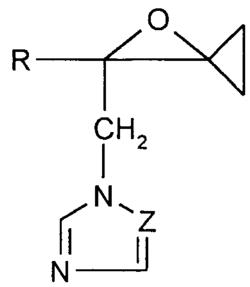

(II)

in welcher

R und Z die oben angegebenen Bedeutungen haben,

mit Natriumazid und in Gegenwart eines zusätzlichen Verdünnungsmittels sowie gegebenenfalls in Gegenwart einer Base umsetzt und unter Zugabe von Wasser aufarbeitet,

und gegebenenfalls anschließend an die so erhaltenen Verbindungen der Formel (I) eine Säure oder ein Metallsalz addiert.

**4.** Fungizide Mittel, gekennzeichnet durch einen Gehalt an mindestens einem Cyclopropyl-ethyl-azol der Formel (I) gemäß Anspruch 1 bzw. an einem Säureadditions-Salz oder Metallsalz-Komplex eines Cyclopropyl-ethyl-azoles der Formel (I).

**5.** Verwendung von Cyclopropyl-ethyl-azolen der Formel (I) gemäß Anspruch 1 bzw. von deren Säureadditions-Salzen und Metallsalz-Komplexen zur Bekämpfung von Pilzen.

**6.** Verfahren zur Bekämpfung von Pilzen, dadurch gekennzeichnet, daß man Cyclopropyl-ethyl-azole der Formel (I) gemäß Anspruch 1 bzw. deren Säureadditions-Salze und Metallsalz-Komplexe auf die Pilze und/oder deren Lebensraum ausbringt.

**7.** Verfahren zur Herstellung von fungiziden Mitteln, dadurch gekennzeichnet, daß man Cyclopropyl-ethyl-azole der Formel (I) gemäß Anspruch 1 bzw. deren Säureadditions-Salze oder Metallsalz-Komplexe mit Streckmitteln und/oder oberflächenaktiven Mitteln vermischt.

**8.** Oxirane der Formel

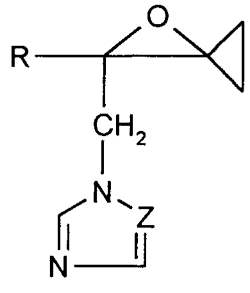

(II)

in welcher

R für Alkyl, Alkenyl, Alkinyl, gegebenenfalls durch Alkyl substituiertes Cycloalkyl, gegebenenfalls substituiertes Aryl, gegebenenfalls substituiertes Aralkyl oder gegebenenfalls substituiertes Aralkenyl steht und

Z für ein Stickstoffatom oder eine CH-Gruppe steht.

**9.** Verfahren zur Herstellung von Oxiranen der Formel

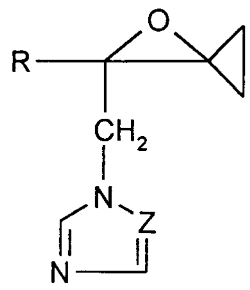

(II)

in welcher

R für Alkyl, Alkenyl, Alkinyl, gegebenenfalls durch Alkyl substituiertes Cycloalkyl, gegebenenfalls substituiertes Aryl, gegebenenfalls substituiertes Aralkyl oder gegebenenfalls substituiertes Aralkenyl steht und

Z für ein Stickstoffatom oder eine CH-Gruppe steht,

dadurch gekennzeichnet, daß man Cyclopropyl-hydroxyethyl-azole der Formel

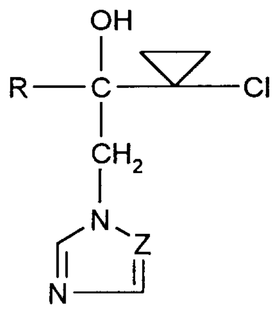

(III)

in welcher

R und Z die oben angegebenen Bedeutungen haben,

in Gegenwart von starken Basen und in Gegenwart eines Verdünnungsmittels umsetzt.

Europäisches Patentamt

# EUROPÄISCHER RECHERCHENBERICHT

Nummer der Anmeldung
EP 95 10 4638

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,Y | EP-A-0 123 160 (IMPERIAL CHEMICAL INDUSTRIES) 31.Oktober 1984<br>* das ganze Dokument *<br>--- | 1,2,4-7 | C07D249/08<br>C07D233/61<br>C07D405/06<br>A01N43/653<br>A01N43/50 |
| D,Y | EP-A-0 180 136 (BAYER AG) 7.Mai 1986<br>* das ganze Dokument *<br>--- | 1,2,4-7 | |
| D,Y | EP-A-0 297 345 (BAYER AG) 4.Januar 1989<br>* das ganze Dokument *<br>--- | 1,2,4-7 | |
| D,Y | EP-A-0 440 949 (BAYER AG) 14.August 1991<br>* das ganze Dokument *<br>--- | 1,2,4-7 | |
| Y | EP-A-0 575 122 (ROHM AND HAAS COMPANY) 22.Dezember 1993<br>* das ganze Dokument *<br>--- | 1,2,4-7 | |
| A | JOURNAL OF ORGANIC CHEMISTRY, Bd. 29, September 1964 Seiten 2588-2592, BURGER A. & ONG H.H. 'Phenylcyclobutane amino alcohols and amino ketones'<br>* Seite 2588; Verbindungen der Formel XVIII *<br>--- | 1 | **RECHERCHIERTE SACHGEBIETE (Int.Cl.6)** |
| | | | C07D<br>A01N |
| A | GB-A-2 136 801 (SANDOZ LTD.) 26.September 1984<br>* das ganze Dokument *<br>--- | 1 | |
| A | JOURNAL OF THE AMERICAN CHEMICAL SOCIETY, Bd. 95, Nr. 16, 8.August 1973 Seiten 5311-5321, TROST B.M. & BOGDANOWICZ M.J. 'New synthetic reactions. Facile synthesis of oxaspiropentanes, versatile synthetic intermediates'<br>* Seite 5312; Tabelle I *<br>---<br>-/-- | 8 | |

Der vorliegende Recherchenbericht wurde für alle Patentansprüche erstellt

| Recherchenort | Abschlußdatum der Recherche | Prüfer |
|---|---|---|
| MÜNCHEN | 7.August 1995 | Hartrampf, G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung
EP 95 10 4638

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
| --- | --- | --- | --- |
| A | EP-A-0 094 564 (BASF AKTIENGESELLSCHAFT)<br>23.November 1983<br>* Anspruch 1 *<br>--- | 8 | |
| A | TETRAHEDRON LETTERS,<br>Bd. 29, Nr. 26, Juli 1988<br>Seiten 3265-3268,<br>KRIEF A. ET AL. 'Exploratory study on .alpha.-metallo selenones'<br>* das ganze Dokument *<br>----- | 8 | |

RECHERCHIERTE SACHGEBIETE (Int.Cl.6)

Der vorliegende Recherchenbericht wurde für alle Patentansprüche erstellt

| Recherchenort | Abschlußdatum der Recherche | Prüfer |
| --- | --- | --- |
| MÜNCHEN | 7.August 1995 | Hartrampf, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument